# EUROPÄISCHE PATENTANMELDUNG

(11) **EP 0 979 806 A1**
(43) Veröffentlichungstag der Anmeldung: **16.02.2000**
(21) Anmeldenummer: 99113318.2
(22) Anmeldetag: 09.07.1999
(51) Int. Cl.: C07B 63/00

(54) **Verfahren zur selektiven Anreicherung und Trennung aromawirksamer Moleküle durch Adsorption**

(30) Priorität: 06.08.1998 DE 19835542
(71) Anmelder: Degussa-Hüls Aktiengesellschaft, 60287 Frankfurt am Main (DE)
(72) Erfinder: Berger, Ralf, Prof.Dr., 30455 Hannover (DE); Krings, Ulrich, Dr., 31628 Landesbergen (DE); Latza, Elke, 30161 Hannover (DE); Treffenfeldt, Wiltrud Prof.Dr., 63179 Obertshausen (DE); Vollmer, Helga, Dr., 63457 Hanau (DE); Preuss, Andrea, Dr., 63456 Hanau (DE)

(57) **Zusammenfassung**

Die Erfindung betrifft ein Verfahren zur Anreicherung, Trennung und Aufreinigung aromawirksamer Moleküle aus wäßrigen oder organischen Lösungen durch Adsorption an zeolithischen Materialien.

Prozeßströme, für die das erfindungsgemäße Verfahren geeignet ist, stammen aus der Herstellung niedermolekularer lipophiler Wertstoffe, insbesondere Aromastoffen, z. B. aus der chemischen Synthese oder aus mikrobiellen bzw. enzymatischen Prozessen. Es handelt sich ebenso um Destillate, Preßsäfte, Homogenate, Mazerate, Lysate oder Suspensionen pflanzlichen Ursprungs.

## Beschreibung

Die Erfindung betrifft ein Verfahren zur Anreicherung, Trennung und Aufreinigung aromawirksamer Moleküle aus wäßrigen oder organischen Lösungen durch Adsorption an zeolithischen Materialien.

Prozeßströme, für die das erfindungsgemäße Verfahren geeignet ist, stammen aus der Herstellung niedermolekularer lipophiler Wertstoffe, insbesondere Aromastoffen, z. B. aus der chemischen Synthese oder aus mikrobiellen bzw. enzymatischen Prozessen. Es handelt sich ebenso um Destillate, Preßsäfte, Homogenate, Mazerate, Lysate oder Suspensionen pflanzlichen Ursprungs.

Bei *L: Janssens et al. (Process Biochemistry 27 (1992) 195-215 und R.G. Berger (Aroma Biotechnology, Springer-Verlag Berlin, Heidelberg (1995)* findet sich ein Überblick über die Herstellung von Aromastoffen durch Mikroorganismen.

Neben den erwünschten Zielsubstanzen enthalten solche Lösungen noch Neben- oder Folgeprodukte, Salze, Zucker, Proteine oder auch Mikroorganismen und sonstige Nebenkomponenten aus der Herstellung und/oder Aufreinigung, z. B. Lösungsmittel. Für die Herstellung einer vertriebsfähigen Form der Zielsubstanzen, ist es notwendig, diese selektiv abzutrennen und zu hohen Reinheitsgraden aufzureinigen. Normalerweise erfolgt diese Aufreinigung mit Hilfe destillativer Trennverfahren oder durch Extraktion (PCT-WO 96/22381).

Die gängigen Verfahren zur Aufreinigung z. B. von Terpenen haben jedoch eine Reihe von Nachteilen:
Destillative Trennverfahren bedingen einen hohen Energieaufwand und können zur thermischen Veränderung der Zielsubstanzen führen. Bei der extraktiven Aufarbeitung von Wertstoffen kommen hohe Mengen ökologisch bedenklicher Lösungsmittel zum Einsatz. Dabei besteht die Gefahr, daß die Zielsubstanzen durch Lösungsmittelreste verunreinigt bleiben oder daß sie mit den Lösungsmitteln reagieren und es dadurch zu Ausbeuteverlusten kommt. Auch die Adsorption von verschiedenen Aromakomponenten an Ionenaustauschern ist bekannt.
   (*A. Klingenberg, J.P. Hanssen, Chem. Biochem. Eng. Q 2 (4) (1988) 222-224)*.

Ionenaustauscher und weitere auch anorganische Adsorbentien für Aromastoffe werden von *U. Krings et al.* beschrieben (J. Chem. Tech. Biotechnol. 1993, (58), 293-299.

Aufgabe der Erfindung ist es, ein Verfahren bereitzustellen, das zur Anreicherung, Trennung und Aufreinigung von kleinen, chemisch und konstitutiv ähnlichen Verbindungen sowie zur Fraktionierung komplexer Substanzgemische, insbesondere aromawirksamer Verbindungen aus wässrigen und/oder organischen Lösungen geeignet ist.

Gegenstand der Erfindung ist ein Verfahren zur Anreicherung, Trennung und Aufreinigung von aromawirksamen Verbindungen durch Adsorption aus wässrigen und/oder organischen Lösungen, das dadurch gekennzeichnet ist, daß man die wäßrigen Lösungen, die eine oder mehrere dieser bevorzugt niedermolekularen unpolaren bis mittelpolaren Verbindungen enthalten, mit einem sauren Zeolith bei einem pH-Wert von 2-12, insbesondere im neutralen oder sauren Bereich in Kontakt bringt, nach erfolgter Adsorption mindestens einer Komponente den Zeolithen abtrennt und diese mit einem geeigneten Lösungsmittel desorbiert. Dadurch werden zwei Lösungen erhalten, die unterschiedliche Komponenten enthalten (Ad- und Desorptionslösung). Zielsubstanzen für die selektive Trennung sind Riech-, Duft- und Aromastoffe, z. B. Terpene und deren Derivate, Lactone, Ester, Aldehyde, Säuren, Alkohole und Ketone. Die Verbindungen dieser chemischen Substanzklassen, die aromawirksam sind, sind dem Fachmann geläufig (s. zitierter Stand der Technik,insbesondere *Janssens et al*.s.o., S. 196,197)

Mit dem erfindungsgemäßen Verfahren können insbesondere lipophile Moleküle, besonders niedermolekulare (Molekülgewicht <300), unpolare bis mittelpolare Verbindungen (Dielektrizitätskonstante <40) abgetrennt und/oder aufgereinigt werden.

Neben der Abtrennung und Aufreinigung einzelner Verbindungen steht die Trennung binärer Aromastoffgemische, z. B. aus der Destillation, in die Einzelverbindungen im Vordergrund.
Dazu gehören insbesondere Terpene wie Limonen/Geraniol, Limonen/Linalool, Myrcen/Myrcenol, α-Pinen/Borneol und Thymol/Menthol.

Besonders geeignet ist das Verfahren für die Trennung von Limonen und Carvon.

Der erfindungsgemäße Einsatz ausgewählter Zeolithe ermöglicht insbesondere eine hochselektive Adsorption bzw. Trennung binärer oder komplex zusammengesetzter Gemische von Verbindungen, die sich nur durch eine funktionelle Gruppe oder Doppelbindung chemisch unterscheiden oder die Konstitutionsisomere sind, und für die bisher kein geeignetes Trennverfahren zur Verfügung steht. Dazu gehört z. B. die Trennung von Limonen und Carvon bzw. die Trennung von 2-Methylbutansäuremethylester und 3-Methylbutansäuremethylester.
Die angesprochenen Gemische stammen im allgemeinen aus der Aufarbeitung natürlicher Rohstoffe, sind aber auch Produkte chemischer Synthesen.
In Abhängigkeit von der zu fordernden Reinheit der gewünschten Produkte ist das Trennverfahren ein- oder mehrstufig durchzuführen.
Die Adsorption kann in einem Temperaturbereich von 0 bis 200 ° C bei pH-Werten zwischen 2 und 12 erfolgen bevorzugt bei 10 bis 60 ° C und bei pH-Werten von 2 bis 8.
Wegen der hohen Flüchtigkeit einiger Verbindungen verwendet man bevorzugt gasdicht verschlossene Gefäße.

Die wäßrigen Lösungen können unterschiedlichen Ursprungs sein. Sie können beispielsweise aus Wasserdampfdestillationen stammen oder Fermentationsbrühen sein, aus denen man bevorzugt zuvor die Biomasse abgetrennt hat.

Es ist aber auch möglich, den als Adsorbens verwendeten Zeolith bereits während der Fermentation zuzusetzen, so kontinuierlich die gewünschte Verbindung durch Adsorption zu entfernen und die Ausbeute an gewünschtem Produkt zu erhöhen.
Die Konzentrationen der aufzureinigenden bzw. voneinander zu trennenden Verbindungen bzw. ihrer Derivate liegen zwischen 1 µg/l und 10 g/l, insbesondere zwischen 1 mg/l und 1 g/l.

Für die Adsorption der erfindungsgemäß abzutrennenden Verbindung sind besonders saure Zeolithe der Typen DAY, Mordenit oder Beta und insbesondere ZSM5 und dealuminierter ZSM5, mit Moduli zwischen 28 und 1000 geeignet.

Die Konzentration des Adsorbens in der Lösung beläuft sich im allgemeinen auf 0,1 bis 800 g/l, insbesondere zwischen 1 bis 200 g/l.
Die einzusetzende Konzentration ist primär von der Kapazität des Adsorbens für das Adsorptiv sowie von der vorliegenden Konzentration des Adsorptivs in der Lösung abhängig.

Zur Adsorption geeignet sind entweder feinkörnige Zeolithpulver der oben genannten Typen mit einem Partikeldurchmesser von 0,1 - 10,0 µm, vorzugsweise von 1 bis 10 µm oder entsprechende Zeolith-Formkörper mit Korngrößen von 0,1 bis 10,0 mm, bevorzugt zwischen 1 und 2 mm.
Die Desorption kann ein- oder mehrstufig mit verschiedenen Lösungsmitteln erfolgen, z.B. mit Methanol, Isopropanol, Ethylacetat und Diethylether oder auch mit Wasser.
Bevorzugt werden Isopropanol, Ethylacetat und Diethylether entsprechend ihrer sinkenden Polarität in mehreren aufeinanderfolgenden Desorptionsstufen eingesetzt.

Dabei beläuft sich die Konzentration des Zeoliths in der (den) Desorptionstufe(n) auf 0,1 bis 800 g/l, bevorzugt auf 1 bis 30 g/l.
Um den neben der primär adsorbierten Verbindung nicht adsorbierten Anteil aus binären oder komplex zusammengesetzen Gemischen gewinnen zu können, benutzt man bevorzugt andere Adsorbentien
z. B. des Typs Lewatit® (organischer Kationenaustauscher) oder anorganische Adsorbentien, insbesondere Zeolithe des Moduls 28 bis 1000.Dabei ist jedoch zu beachten, daß sich diese im allgemeinen von denen unterscheiden, die zur Abtrennung der Hauptkomponente eingesetzt werden.
Die Adsorbentien werden dann analog zu dem oben beschriebenen Verfahren aufgearbeitet.

Für die verfahrenstechnische Realisierung stehen das Batch-, Festbett oder Crossflow-Filtrationsverfahren, kontinuierlich oder diskontinuierlich durchgeführt, zur Verfügung.

In einer vorteilhaften Ausführungsform kombiniert man die erfindungsgemäße Adsorption mit einer Crossflow-Filtration, bei der man
a) die beladenen Zeolithe in Form von Suspensionen an einer porösen Fläche/Membran vorbeiströmen läßt, und gleichzeitig
b) eine Druckdifferenz zwischen der Überström- und der gegenüberliegenden Seite der Fläche/Membran einstellt, so daß
c) ein Teil der die Fläche/Membran überströmenden von den adsorbierten Verbindungen ganz oder teilweise befreiten Lösung die Fläche/Membran quer zur Fließrichtung durchströmt (Filtratfluß)
d) in einem Waschschritt die von den adsorbierten Verbindungen zumindest teilweise befreite Suspension abtrennt,
e) anschließend die adsorbierten Verbindungen aus dem beladenen Zeolith desorbiert und
f) gegebenfalls die die nicht adsorbierten, aber auch noch gewünschte Verbindungen enthaltenden Lösungen aufarbeitet.

Diese Aufarbeitung umfaßt die Isolierung von in dem (den) ersten Adsorptionsschritten nicht erfaßten gewünschten Verbindungen.

Zur Erhöhung der Ausbeute schleust man die Lösungen ein- oder mehrfach in das erfindungsgemäße Reinigungsverfahren ein. Eine Variante des erfindungsgemäßen Verfahrens besteht darin, neben der Verwendung der genannten Zeolithe dieses mit aus dem Stand der Technik bekannten Reinigungsverfahren durch Verwendung von organischen Kationenaustauschern und anderen Reinigungsschritten zu kombinieren.

Ein entsprechender Adsorptions- und Desorptionsschritt erfolgt vor und/oder nach einer Reinigungsstufe durch Adsorption an einem der erfindungsgemäß verwendeten Zeolithe, der sich eine Desorption angeschlossen hat. Das Desorptionsverhalten von organischen Verbindungen ist dem Fachmann bekannt. Die geeigneten Bedingungen werden den chemischen Eigenschaften der Verbindungen eingehalten.

### Beispiel 1

### Trennung von Limonen und Carvon

Für die Trennung von Limonen und Carvon geeignet sind vor allem Zeolithe in Form von Pulvern oder Formkörpern des Typs ZSM5 mit einem Modul zwischen 150 und 1000. Für diese Trennungsaufgabe sind Y-Zeolithe nicht geeignet. Die terpenhaltige Lösung mit Konzentrationen an Limonen und Carvon von jeweils einige hundert mg/l wird ohne vorherige pH-Einstellung mit dem Zeolithen in Kontakt gebracht. Die Konzentration des Adsorbens in der Lösung beträgt ca. 5 g/l. Die Adsorption findet in einem gasdicht verschlossenen Gefäß bei 15 bis 40 ° C im Verlauf von zwei Stunden statt. Das enthaltene Carvon verbleibt in der Lösung. Zur Desorption des adsorbierten Limonens wird das Adsorbens mit Lösungsmitteln, bevorzugt in drei Schritten mit Isopropanol, Ethylacetat und Diethylether drei Minuten lang eluiert. Dabei beträgt die Konzentration des Zeoliths in der Desorptionslösung jeweils ca. 25 g/l.

Aufgrund der besonders hohen Flüchtigkeit des Carvons ist es zweckmäßig, für die Aufarbeitung der wäßrigen Phase ein organisches Adsorbens, z. B. Lewatit® einzusetzen. Hierzu wird nach der Adsorption der Überstand dekantiert, mit ca. 2,5 g/l Lewatit® versetzt und nach zwei Stunden erneut dekantiert. Mit Lewatit® und anderen unspezifischen Adsorbentien, z. B. Polystyrolharz kann das nicht adsorbierte Carvon quantitativ aus der wäßrigen Phase entfernt werden.

### Beispiel 2

### Trennung von 2-Methylbutansäuremethylester und 3-Methylbutansäuremethylester

Für die Trennung von 2-Methylbutansäuremethylester und 3-Methylbutansäuremethylester ist insbesondere der Zeolith ZSM-5 mit Modul 150 geeignet. Die wäßrige Lösung der Ester mit Konzentrationen von jeweils ca. 100 mg/l wird bei neutralem pH-Wert mit dem Zeolithen in Kontakt gebracht. Die Konzentration des Adsorbens in der Lösung beträgt ca. 2 g/l. Die Adsorption findet in einem gasdicht verschlossenem Gefäß bei 15 bis 30 ° C im Verlauf von 10 Minuten statt. 3-Methylbutansäuremethylester wird vollständig adsorbiert, 2-Methylbutansäuremethylester verbleibt zu ca. 50 % in der wäßrigen Lösung. Der Zeolith wird abgetrennt. Für die Aufarbeitung der den restlichen Anteil enthaltenden wäßrigen Phase wird 1 g/l Zeolith ZSM-5, Modul 1000 zugegeben und nach ca. 30 Minuten abgetrennt. Der adsorbierte 2-Methylbutansäuremethylester wird daraus dreimal mit Ethylacetat drei Minuten lang eluiert. Dabei beträgt die Konzentration des Zeoliths in der Desorptionslösung jeweils ca. 25 g/l.
Das an ZSM5 Modul 150 adsorbierte, mit 3-Methylbutansäuremethylester angereicherte Estergemisch wird ebenso desorbiert und einem oder mehreren weiteren Aufreinigungsschritten zugeführt.

## Patentansprüche

1. Verfahren zur Anreicherung, Trennung und Aufreinigung von aromawirksamen Verbindungen durch Adsorption aus wäßrigen und/oder organischen Lösungsmitteln enthaltenden Lösungen,
**dadurch gekennzeichnet,**
daß man die Lösungen dieser Verbindungen ein- oder mehrfach mit einem sauren Zeolith in Kontakt bringt, der einen Modul von 28 bis 1000 besitzt, nach erfolgter Adsorption den bevorzugt keine oder eine oder mehrere der nicht adsorbierten Verbindungen enthaltenden Überstand abtrennt und die adsorbierte Verbindung mit einem Lösungsmittel von dem eingesetzten Adsorbens desorbiert.

2. Verfahren gemäß Anspruch 1,
**dadurch gekennzeichnet,**
daß man den nach der Adsorption erhaltenen Überstand anschließend mit einem weiteren sauren Zeolith der gegebenenfalls einen anderen Modul als der im ersten Adsorptionsschritt verwendeten Zeolith aufweist in Kontakt bringt, das Adsorbens abtrennt und die adsorbierte Verbindung nach der Desorption isoliert.

3. Verfahren gemäß Anspruch 1 und 2,
**dadurch gekennzeichnet,**
daß man Lösungen aus mikrobiellen oder enzymatischen Prozessen, eine Fermentationslösung, Lösungen aus der Biotransformation oder Biokatalyse oder Lösungen pflanzlichen Ursprungs, z. B. Destillate, Preßsäfte, Homogenate, Mazerate, Lysate und Suspensionen einsetzt.

4. Verfahrem gemäß Anspruch 3,
**dadurch gekennzeichnet,**
daß man vor der Adsorption die Mikroorganismen oder Feststoffanteile zumindest teilweise aus der Fermentationslösung abtrennt.

5. Verfahren gemäß den Ansprüchen 1 bis 4,
**dadurch gekennzeichnet**,
daß man vor der Adsorption die löslichen Proteine zumindest teilweise aus der Fermentationslösung abtrennt.

6. Verfahren gemäß Anspruch 1,
**dadurch gekennzeichnet,**
daß man eine aus der chemischen Synthese gewonnene Lösung einsetzt.

7. Verfahren gemäß einem oder mehreren der Ansprüche 1 bis 6,
**dadurch gekennzeichnet**,
daß man als Adsorbentien Zeolithe der Typen DAY, ZSM-5, oder dealuminierten ZSM-5 mit den Moduli von 28 bis 1000 einsetzt.

8. Verfahren gemäß Anspruch 10,
**dadurch gekennzeichnet**,
daß man Zeolithe vom Typ ZSM5 oder dealuminierten ZSM-5 in der H-, Ammonium- oder Na-Form einsetzt.

9. Verfahren gemäß einem oder mehreren der Ansprüche 1 bis 8 zur Trennung und Aufreinigung,
**dadurch gekennzeichnet**,
daß man es mit einer Crossflow-Filtration kombiniert, wobei man
a) die beladenen Zeolithe in Form von Suspensionen an einer porösen Fläche/Membran vorbeiströmen läßt, wobei man
b) eine Druckdifferenz zwischen der Überström- und der gegenüberliegenden Seite der Fläche/Membran einstellt, so daß
c) ein Teil der die Fläche/Membran überströmenden von den adsorbierten Verbindungen ganz oder teilweise befreiten die nicht adsorbierten Verbindungen enthaltenden Lösung die Fläche/Membran quer zur Fließrichtung durchströmt (Filtratfluß)
d) in einem Waschschritt die von den adsorbierten Verbindungen befreite Lösung abtrennt, und
e) anschließend die adsorbierten Verbindungen von dem Adsorbens desorbiert.

10. Verfahren gemäß Anspruch 9,
**dadurch gekennzeichnet,**
daß man Zeolithe mit einem mittleren Partikeldurchmesser von 0,1 bis 10, µm oder Formkörper mit einem Durchmesser von 0,1 bis 100 mm einsetzt.

11. Verfahren gemäß den Ansprüchen 9 und 10,
**dadurch gekennzeichnet,**
daß man einen Transmembrandruck von 0,1 bis 3 bar einstellt.

12. Verfahren gemäß den Ansprüchen 9 bis 11,
**dadurch gekennzeichnet,**
daß man keramische oder organische Membranen/poröse Flächen mit Ultrafiltrations- oder Mikro- oder Nanofiltrationseigenschaften verwendet.

13. Verfahren gemäß einem oder mehreren der vorhergehenden Ansprüche,
**dadurch gekennzeichnet,**
daß man die desorbierte Lösung ein- oder mehrfach der Adsorption und Desorption unterwirft.

14. Verfahren gemäß einem oder mehreren der vorhergehenden Ansprüche,
**dadurch gekennzeichnet,**
daß man den Überstand aus der Adsorption an Zeolithen mit Kationenaustauschern in Kontakt bringt oder anderen Reinigungsschritten kombiniert und die gewünschten Verbindungen anschließend isoliert.

15. Verfahren gemäß einem oder mehreren der vorhergehenden Ansprüche,
**dadurch gekennzeichnet,**
man Gemische aus Limonen/Geraniol oder Limonen/Linalool auftrennt.

16. Verfahren gemäß einem oder mehreren der vorhergehenden Ansprüche,
**dadurch gekennzeichnet,**
daß man ein Gemisch aus Myrcen/Myrcenol oder α-Pinen/Borneol oder Thymol/Menthol auftrennt.

17. Verfahren gemäß einem oder mehreren der vorhergehenden Ansprüche,
**dadurch gekennzeichnet,**
daß man Gemische von Limonen und Carvon auftrennt.

18. Verfahren gemäß einem oder mehreren der vorhergehenden Ansprüche,
**dadurch gekennzeichnet,**
daß man Gemische aus 2-Methylbutansäuremethylester und 3- Methylbutansäuremethylester auftrennt.
